# EUROPEAN PATENT APPLICATION

(11) **EP 4 361 618 A1**
(43) Date of publication of application: **01.05.2024**
(21) Application number: 22828502.9
(22) Date of filing: 23.06.2022
(51) Int. Cl.: G01N 27/327, G01N 27/416

(54) **ENZYME IMMOBILIZED ELECTRODE, ENZYME IMMOBILIZED ELECTRODE MANUFACTURING METHOD, TARGET MOLECULE OXIDATION-REDUCTION METHOD, AND TARGET MOLECULE OXIDATION-REDUCTION DEVICE**

(30) Priority: 23.06.2021 JP 2021103850
(71) Applicant: Panasonic Intellectual Property Management Co., Ltd., Kadoma-shi, Osaka 571-0057 (JP)
(72) Inventor: WAYAMA, Fumiya, Kadoma-shi, Osaka 571-0057 (JP); HATSUGAI, Noriyuki, Kadoma-shi, Osaka 571-0057 (JP); OKUMURA, Yasuaki, Kadoma-shi, Osaka 571-0057 (JP)
(74) Representative: Novagraaf International SA
(86) International application number: PCT/JP2022/025122
(87) International publication number: WO 2022/270586

(57) **Abstract**

An immobilized enzyme electrode includes: an electrode; a redox enzyme that oxidizes or reduces a target molecule; and an electron carrier that performs electron transport between the electrode and the redox enzyme. The electron carrier is immobilized on the electrode via a first linker that is in a form of a chain, and the redox enzyme is immobilized on the electrode via a second linker that is longer than the first linker, the second linker being in a form of a chain.

## Description

### [Technical Field]

The present disclosure relates to an immobilized enzyme electrode, an immobilized enzyme electrode manufacturing method, a target molecule redox method, and a target molecule redox device.

### [Background Art]

As methods for immobilizing enzymes on an electrode, there is a method in which enzymes are immobilized on a surface of an electron using a chemical bond, and a method in which enzymes are comprehensively immobilized using polymer. For example, Patent Literature (PTL) 1 discloses an enzyme electrode obtained by immobilizing a carrier, an enzyme, and a mediator on a conductive component.

### [Citation List]

### [Patent Literature]

[PTL 1] Japanese Unexamined Patent Application Publication No. 2006-058289

### [Summary of Invention]

### [Technical Problem]

However, the enzyme electrode disclosed in Patent Literature (PTL) 1 can be used as a sensor for observing electron transfer from the enzyme to the electrode and measuring the current value, but cannot supply an electron from the electrode to the enzyme and further from the enzyme to a substrate (hereinafter also referred to as a target molecule) in a sample.

In view of this, the present disclosure provides an immobilized enzyme electrode, and immobilized enzyme electrode manufacturing method, a target molecule redox method, and a target molecule redox device that are capable of transporting electrons from the electrode to a redox enzyme, and, in addition, transport electrons from the redox enzyme to a target molecule in a sample.

### [Solution to Problem]

An immobilized enzyme electrode according to an aspect of the present disclosure includes: an electrode; a redox enzyme that oxidizes or reduces a target molecule; and an electron carrier that donates an electron received from the electrode to the redox enzyme, wherein the electron carrier is immobilized on the electrode via a first linker that is in a form of a chain, and the redox enzyme is immobilized on the electrode via a second linker that is longer than the first linker, the second linker being in a form of a chain.

Furthermore, an immobilized enzyme electrode manufacturing method according to an aspect of the present disclosure is an immobilized enzyme electrode manufacturing method for manufacturing the above-described immobilized enzyme electrode and includes: immobilizing the electron carrier on the electrode via the first linker that is in a form of a chain; and immobilizing the redox enzyme on the electrode via a second linker.

Furthermore, a target molecule redox method according to an aspect of the present disclosure includes: oxidizing or reducing a target molecule using an immobilized enzyme electrode manufactured according to the above-described immobilized enzyme electrode manufacturing method.

Furthermore, a target molecule redox device according to an aspect of the present disclosure includes: the above-described immobilized enzyme electrode; and a power supply that applies voltage to the immobilized enzyme electrode, wherein the target molecule redox device oxidizes or reduces a target molecule.

### [Advantageous Effects of Invention]

The present disclosure can provide an immobilized enzyme electrode, and immobilized enzyme electrode manufacturing method, a target molecule redox method, and a target molecule redox device that are capable of transporting electrons from the electrode to a target molecule in a sample.

### [Brief Description of Drawings]

[FIG. 1]

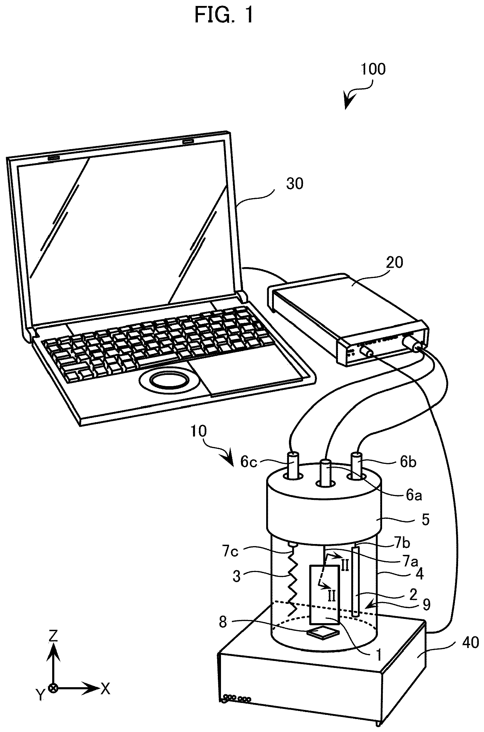

   FIG. 1 is a diagram illustrating an example of a configuration of a target molecule redox device including an immobilized enzyme electrode according to an embodiment.
[FIG. 2]

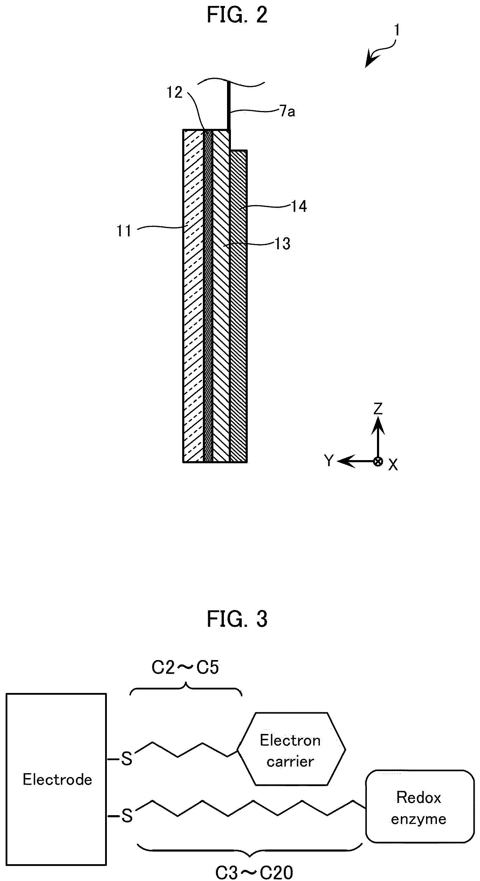

   FIG. 2 is a cross-sectional view taken at line II-II in FIG. 1.
[FIG. 3]
   FIG. 3 is a diagram schematically illustrating an electron carrier and a redox enzyme that are immobilized on an electrode.
[FIG. 4]

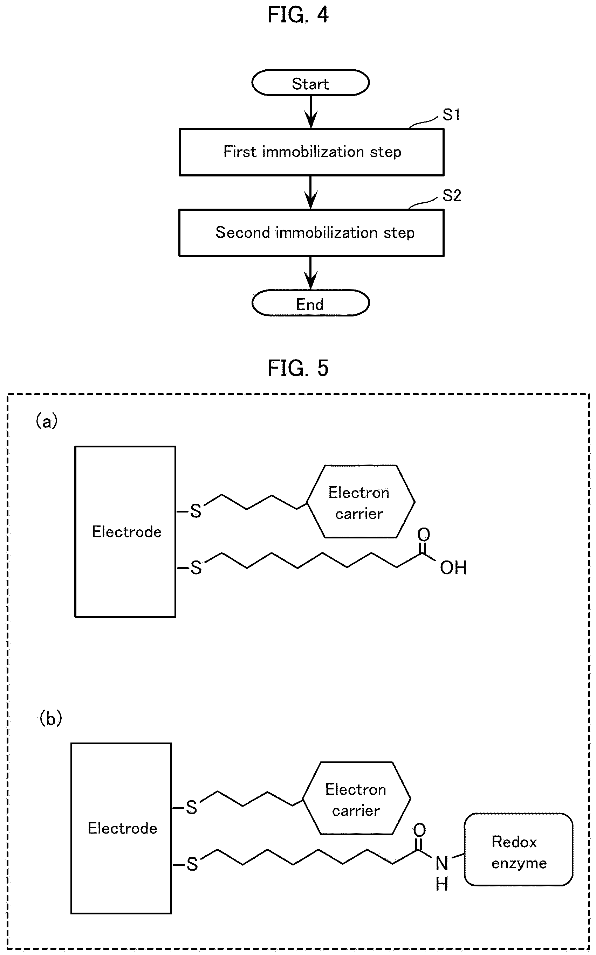

   FIG. 4 is a flowchart illustrating an example of an immobilized enzyme electrode manufacturing method.
[FIG. 5]
   FIG. 5 is a diagram schematically illustrating respective flows in FIG. 4.
[FIG. 6]

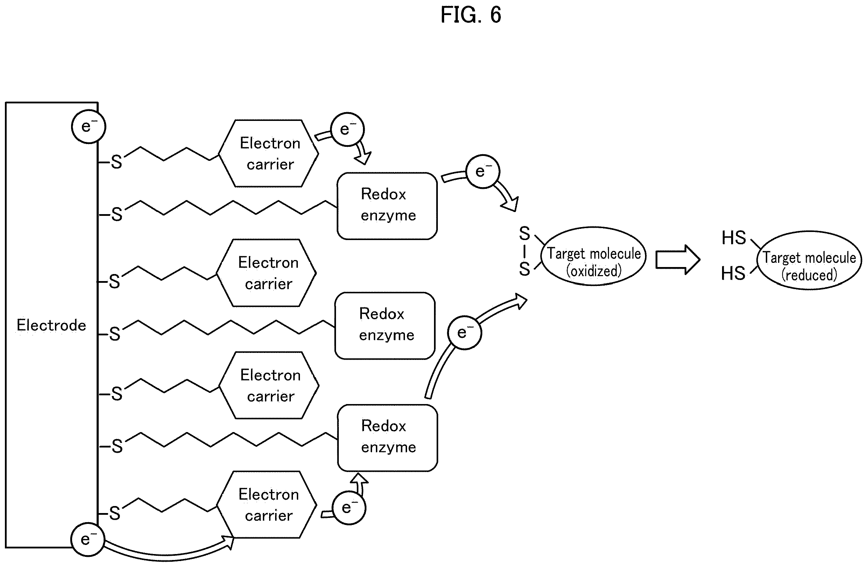

   FIG. 6 is diagram for describing a target molecule redox method.
[FIG. 7]

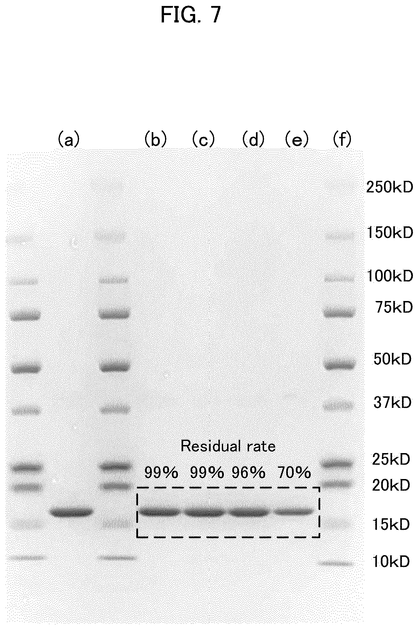

   FIG. 7 is a diagram illustrating electrophoretic patterns after SDS-PAGE for Working Example 1, Comparative Example 1, Comparative Example 2, and Comparative Example 3.
[FIG. 8]

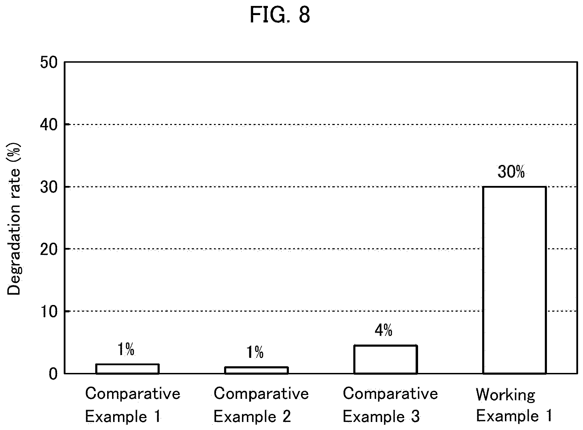

   FIG. 8 is a graph illustrating target molecule degradation rates for Working Example 1, Comparative Example 1, Comparative Example 2, and Comparative Example 3.
[FIG. 9]

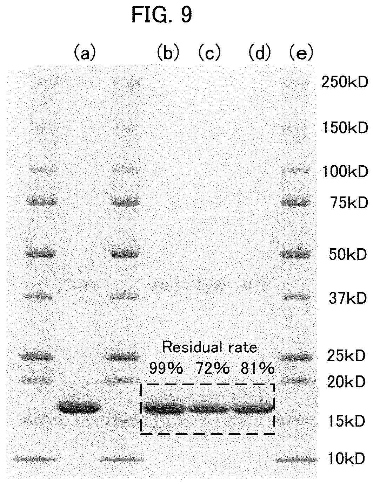

   FIG. 9 is a diagram illustrating electrophoretic patterns after SDS-PAGE for Working Example 1, Comparative Example 4, and Comparative Example 5.
[FIG. 10]

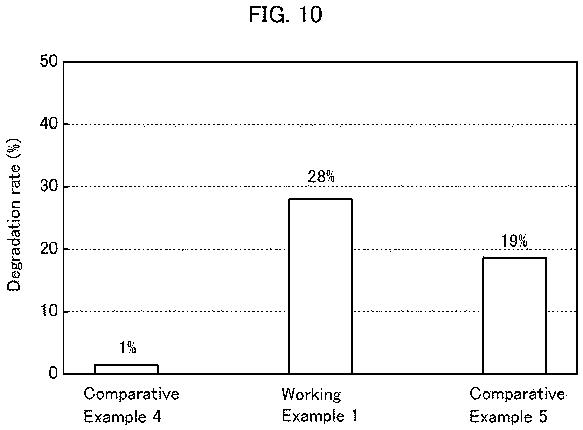

   FIG. 10 is a graph illustrating target molecule degradation rates for Working Example 1, Comparative Example 4, and Comparative Example 5.

### [Description of Embodiments]

### (Underlying Knowledge Leading to Present Disclosure)

An immobilized enzyme electrode in which an enzyme is immobilized on an electrode has conventionally been used in a biosensor and the like. However, the immobilized enzyme electrode is used to measure electron transfer from the enzyme to the electrode, but cannot realize electron transport from the electrode to the enzyme, and cannot realize electron transport from the enzyme to a target molecule, either. The electron transport from the electrode to the enzyme is difficult because the enzyme forms a complicated three-dimensional structure that makes it difficult to accept an electron, or because when the enzyme is too close to the electrode, the structure is easily broken.

As a method for immobilizing an enzyme on an electrode, there is a method in which an electron transfer substance and an enzyme are entrapped in a polymer and immobilized on an electrode, and the immobilized enzyme electrode manufactured by the method has low reactivity with the substrate (target molecule). This is because when an electron is transported to the enzyme through electron transfer, the enzyme does not exist on the uppermost surface of the immobilized enzyme electrode, or the enzyme is entrapped in the polymer and thus has no degree of freedom.

For this reason, there is a demand for an immobilized enzyme electrode that allows the enzyme to accept an electron from the electrode and that can supply an electron to a substrate in a sample (in other words, the substrate has good reactivity). Examples of the enzyme used in the immobilized enzyme electrode include a redox enzyme. A redox enzyme is required to oxidize or reduce a substrate in a sample (so-called target molecule). In order for the redox enzyme immobilized on the electrode to act on the substrate, an electron needs to be transported from the electrode to the redox enzyme. An electron is usually transported from the electrode to the enzyme using an electrochemical measurement device or the like. At this time, an electron transfer substance such as a mediator or the like is used in order to transport an electron more efficiently. It is not preferable to mix an enzyme and an electron transfer substance into a sample, and thus there is a demand for use of an immobilized enzyme electrode in which these substances are immobilized on an electrode.

Hence, the present inventors have carried out an intensive study in view of the above problems and as a result have found an immobilized enzyme electrode in which an electron carrier and an enzyme are each immobilized on an electrode by a chemical bond, and in which the electron carrier is immobilized on the electrode by a first linker having conductivity and the enzyme is immobilized on the electrode by a second linker having no conductivity. The immobilized enzyme electrode is characterized that the first linker and the second linker are in the form of a chain, the second linker is longer than the first linker, and a distance between the electron carrier and the enzyme immobilized on the electrode by these linkers is small (for example, within several µm) enough to allow an electron to move. In the immobilized enzyme electrode according to the present disclosure, both the electron carrier (for example, mediator) and the enzyme are immobilized on the electrode, and the distance between the electron carrier and the enzyme is small enough to allow electron transfer, and thus it is possible to realize electron transport from the electrode to the redox enzyme and also electron transport from the redox enzyme to a substrate (so-called target molecule) in a sample.

### (One aspect of the present disclosure)

An outline of an aspect of the present disclosure is as follows.

An immobilized enzyme electrode according to an aspect of the present disclosure includes: an electrode; a redox enzyme that oxidizes or reduces a target molecule; and an electron carrier that provides an electron received from the electrode to the redox enzyme. Here, the electron carrier is immobilized on the electrode via a first linker that is in a form of a chain, and the redox enzyme is immobilized on the electrode via a second linker that is longer than the first linker, the second linker being in a form of a chain.

Accordingly, with the immobilized enzyme electrode, electrons donated from the electrode to the electron carrier can be easily transported to the redox enzyme. Furthermore, with the immobilized enzyme electrode, the redox enzyme is immobilized on the electrode via the second linker that is in the form of a chain, and thus degree of freedom of the redox enzyme is increased. In addition, with the immobilized enzyme electrode, the redox enzyme is located at the uppermost surface of the immobilized enzyme electrode, and thus electrons accepted by the redox enzyme can be easily transported from the redox enzyme to a target molecule in a sample. Therefore, the immobilized enzyme electrode can transport the electrons from the electrode to the redox enzyme, and in addition, can transport electrons from the redox enzyme to a target molecule in a sample.

For example, in the immobilized enzyme electrode according to an aspect of the present disclosure, the first linker includes an alkyl chain having at least 2 and at most 5 carbon atoms.

Accordingly, since the first linker can be made conductive, electrons donated from the electrode can be easily transported to the electron carrier.

For example, in the immobilized enzyme electrode according to an aspect of the present disclosure, the second linker includes an alkyl chain having at least 3 and at most 20 carbon atoms.

Accordingly, since the second linker can be made to have poor conductivity, transporting of electrons from the electron carrier to the redox enzyme becomes easy.

For example, in the immobilized enzyme electrode according to an aspect of the present disclosure, the electron carrier has a molecular weight of at most 500.

When the molecular weight of the electron carrier exceeds 500, the electron carrier cannot approach the active portion of the redox enzyme, and thus electrons cannot be donated. For this reason, when the molecular weight of the electron carrier is at most 500, the electron carrier is able to donate electrons to the redox enzyme.

Furthermore, an immobilized enzyme electrode manufacturing method according to an aspect of the present disclosure is an immobilized enzyme electrode manufacturing method for manufacturing the above-described immobilized enzyme electrode and includes: immobilizing the electron carrier on the electrode via the first linker that is in a form of a chain; and immobilizing the redox enzyme on the electrode via a second linker.

Accordingly, with the immobilized enzyme electrode manufacturing method, it is possible to manufacture an immobilized enzyme electrode that can transport electrons from the electrode to the redox enzyme, and, in addition, transport electrons from the redox enzyme to a target molecule in a sample.

Furthermore, a target molecule redox method according to an aspect of the present disclosure includes: oxidizing or reducing a target molecule using an immobilized enzyme electrode manufactured according to the above-described immobilized enzyme electrode manufacturing method.

Accordingly, with the target molecule redox method, electrons are transported from the immobilized enzyme electrode to the target molecule, and thus the target molecule can be efficiently oxidized or reduced.

Furthermore, a target molecule redox method according to an aspect of the present disclosure, the target molecule is a protein having a disulfide bond.

Accordingly, with the target molecule redox method, the target molecule can be reduced by reducing the disulfide bond inside the target molecule.

Furthermore, a target molecule redox device according to an aspect of the present disclosure includes: the above-described immobilized enzyme electrode; and a power supply that applies voltage to the immobilized enzyme electrode. Here, the target molecule redox device oxidizes or reduces a target molecule.

Accordingly, with the target molecule redox device, electrons are transported from the immobilized enzyme electrode to the target molecule, and thus the target molecule can be efficiently oxidized or reduced.

It should be noted that these general or specific aspects may be implemented as a system, a method, an apparatus, an integrated circuit, a computer program, or a computer-readable recording medium such as a CD-ROM, or may be implemented as any combination of a system, a method, an apparatus, an integrated circuit, a computer program, and a recording medium.

Hereinafter, embodiments will be described in detail with reference to the drawings.

It should be noted that each of the embodiments described shows a general or specific example of the present disclosure. The numerical values, shapes, materials, elements, the arrangement and connection of the elements, steps, the processing order of the steps, etc., indicated in the following embodiments are mere examples, and thus are not intended to limit the claims. Furthermore, among the elements described in the following embodiments, elements not recited in any one of the independent claims that indicate the broadest concepts are described as optional elements. Furthermore, the figures are schematic illustrations and are not necessarily accurate depictions. Elements which are substantially the same have the same reference signs in the figures, and duplicate description may be omitted or simplified.

The mutually orthogonal X-axis, Y-axis, and Z-axis directions illustrated in the figures will be used as appropriate in the description. In particular, the positive side in the Z-axis direction may be described as the upper side, and the negative side in the Z-axis direction may be described as the lower side.

In the present disclosure, terms indicating relationships between elements such as "parallel" and "perpendicular", terms indicating shapes of elements such as "rectangular", and numerical ranges refer not only to their strict meanings, but encompass a range of essentially equivalents, such as a range of deviations of a few percent.

In the figures of the present disclosure, dashed lines indicate the boundaries of what is not visible from the surface, as well as regions.

### [Embod iment]

Hereinafter, embodiments will be described in detail with reference to FIG. 1 to FIG. 6.

### [Target molecule redox device]

### [1. Outline]

First, an outline of a target molecule redox device according to an embodiment will be described with reference to FIG. 1. FIG. 1 is a diagram illustrating an example of a configuration of a target molecule redox device including an immobilized enzyme electrode according to the present embodiment.

Target molecule redox device 100 performs electron transport between an electrode and a redox enzyme by applying a voltage to the electrode (so-called immobilized enzyme electrode). For example, target molecule redox device 100 transports an electron from the electrode to an electron carrier immobilized on the surface of the electrode, and further transports an electron from the electron carrier to the redox enzyme located on the uppermost surface of the electrode. Accordingly, electron transfer occurs between the immobilized enzyme electrode and the target molecule in the sample, and thus the target molecule is oxidized or reduced. For example, target molecule redox device 100 applies a voltage to the electrode while a sample (for example, sample solution) containing a target molecule is in a non-flowing state, and thereby electron transfer between the redox enzyme immobilized on the electrode, and the target molecule in the sample to oxidize or reduce the target molecule. The oxidized or reduced target molecule is then diffused into the liquid by switching the liquid from the non-flowing state to a flowing state. By repeatedly switching the flow state of the liquid as described above, the target molecule can be efficiently oxidized or reduced throughout the entire liquid.

As used herein, a non-flowing state of a liquid means, for example, that the liquid is not agitated or shaken (for example, not subjected to an external force such as a shearing force or a vibration) and that no fluctuation or other motion is observed on the liquid surface.

### [2. Configuration]

Next, the configuration of target molecule redox device 100 in this embodiment will be described with reference to FIG. 1 to FIG. 3.

As illustrated in FIG. 1, target molecule redox device 100 includes agitator 40 that agitates sample 9 containing a target molecule to bring the sample into a flowing state, an immobilized enzyme electrode (cathode electrode 1) on which a redox enzyme that oxidizes or reduces the target molecule by electron transfer with the target molecule is immobilized, power supply 20 that applies a voltage to the immobilized enzyme electrode, and controller 30 that controls power supply 20 and agitator 40. An electron carrier and the redox enzyme are immobilized on the immobilized enzyme electrode.

Voltage applier 10 is, for example, a three-electrode cell that includes cathode electrode 1 (also referred to as a working electrode), reference electrode 2, counter electrode 3, cell 4, lid 5, terminals 6a, 6b, and 6c, and leads 7a, 7b, and 7c. Voltage applier 10 may be a two-electrode cell that includes, for example, a working electrode (cathode electrode 1) and counter electrode 3.

Cathode electrode 1 and counter electrode 3 are made of a conductive material. The conductive material may be, for example, a carbon material, a conductive polymer material, a semiconductor, or a metal.

First, cathode electrode 1 will be described with reference to FIG. 2 and FIG. 3. FIG. 2 is a cross-sectional view taken at line II-II in FIG. 1. FIG. 3 is a diagram schematically illustrating an electron carrier and a redox enzyme that are immobilized on an electrode.

Cathode electrode 1 is an immobilized enzyme electrode. Cathode electrode 1 includes, for example, glass substrate 11, titanium deposition layer 12 deposited on glass substrate 11, cathode substrate 13 formed on titanium deposition layer 12, and reaction layer 14 including the electron carrier and the redox enzyme that are immobilized on cathode substrate 13.

As cathode substrate 13, for example, a conductive substrate such as gold, glassy carbon, or ITO (Indium Tin Oxide) may be used. The thickness of cathode substrate 13 is not particularly limited.

The electron carrier immobilized on cathode substrate 13 is not particularly limited as long as it is a substance that enables electron transfer between the electrode and the redox enzyme immobilized on the electrode. Examples thereof include a viologen, a quinone, or an indophenol. The electron carrier is immobilized on cathode substrate 13 by a first linker in the form of a chain. The first linker includes, for example, an alkyl chain having at least 2 and at most 5 carbon atoms. The first linker has a thiol group at an end thereof. The electron carrier is immobilized on the surface of the electrode by chemical bonding between the first linker and the electrode (specifically, cathode substrate 13).

The electron carrier and the redox enzyme immobilized on the electrode may have the following properties (1) to (4). (1) The first linker that connects the electron carrier and the electrode has conductivity, and (2) the second linker that connects the redox enzyme and the electrode has a structure similar to that of the first linker but has no conductivity. In addition, (3) the distance between the redox enzyme and the electrode is larger than the distance between the electron carrier and the electrode. (4) The distance between the electron carrier and the redox enzyme is a distance that allows electron transfer (for example, within several µm). Accordingly, an electron transferred from the electrode to the electron carrier can easily move to the redox enzyme. In addition, the redox enzyme is disposed on the uppermost surface of the electrode and is immobilized on the electrode by the second linker in the form of a chain, and thus the redox enzyme has a higher degree of freedom and more easily acts on the target molecule.

The numbers of carbon atoms of the first linker and the second linker may be changed within the above respective preferable ranges depending on the combination of the electron carrier and the redox enzyme.

The second linker having no conductivity includes a case where the second linker conducts electricity slightly but has sufficiently lower conductivity than the first linker and can be regarded as substantially non-conductive.

In addition, the redox enzyme immobilized on the surface (so-called cathode substrate 13) of the electrode is an enzyme that oxidizes or reduces the target molecule. The redox enzyme is immobilized on cathode substrate 13 by the second linker in the form of a chain longer than the first linker. The second linker includes, for example, an alkyl chain having at least 3 and at most 20 carbon atoms. In particular, the second linker may include an alkyl chain having at least 6 and at most 14 carbon atoms.

Reference electrode 2 is an electrode that does not react with the components in sample 9 and maintains a constant potential, and is used to control the potential difference between cathode electrode 1 and reference electrode 2 to a constant level by power supply 20. Reference electrode 2 is, for example, a silver/silver chloride electrode. Counter electrode 3 is, for example, a platinum electrode.

Power supply 20 applies a voltage between cathode electrode 1 and counter electrode 3 of voltage applier 10 to control the potential between cathode electrode 1 and reference electrode 2 to a predetermined value in accordance with a control signal output from controller 30.

Controller 30 processes information for controlling the application of a voltage by power supply 20 and the movement of a motor (not illustrated) of agitator 40. Controller 30 is realized, for example, by a processor, a microcomputer, or dedicated circuitry.

Agitator 40 controls the rotation speed and the rotation time of agitating element 8 that is set in voltage applier 10 by controlling the operation of the motor in accordance with a control signal output from controller 30.

### [3. Immobilized enzyme electrode manufacturing method]

Next, an immobilized enzyme electrode manufacturing method will be described with reference to FIG. 4 and FIG. 5. FIG. 4 is a flowchart illustrating an example of an immobilized enzyme electrode manufacturing method. FIG. 5 is diagrams schematically illustrating flows in FIG. 4. (a) in FIG. 5 illustrates a first immobilization step, and (b) in FIG. 5 illustrates a second immobilization step. The immobilized enzyme electrode is cathode electrode 1.

The immobilized enzyme electrode manufacturing method includes a first immobilization step (S1) and a second immobilization step (S2) of immobilizing the redox enzyme on the electrode via the second linker.

In the first immobilization step (S1), the electron carrier is immobilized on the electrode via the first linker in the form of a chain. First, a solution containing the electron carrier (for example, electron mediator) bonded to the first linker and the second linker is provided, and the first linker bonded to the electron carrier and the second linker are immobilized on the electrode in the same manner as in the formation of a self-assembled monolayer (SAM). For example, the first linker bonded to the electron carrier and the second linker are each mixed into a solution with ethanol:acetonitrile = 1:1, and the electrode is immersed in this mixed solution for 1 hour or more and allowed to stand. As illustrated in (a) in FIG. 5, the electron carrier has the first linker. The first linker has a thiol group at an end thereof. By chemical bonding between the first linker and the electrode, the electron carrier is immobilized on the electrode via the first linker. The first linker is an alkyl chain having a length that allows an electron to move from the electrode to the electron carrier. The first linker is, for example, an alkyl chain having at least 2 and at most 5 carbon atoms.

The second linker for immobilizing the redox enzyme to the electrode is an alkyl chain having at least 3 carbon atoms, and may be an alkyl chain having at least 6 carbon atoms in order to prevent an electron from moving from the electrode directly to the redox enzyme. In addition, the second linker is an alkyl chain having at most 20 carbon atoms, and may be an alkyl chain having at most 14 carbon atoms in order to allow an electron to easily move from the electron carrier bonded to the first linker to the redox enzyme. That is, the second linker has at least 3 and at most 20 carbon atoms, and more preferably, for example, the second linker may be an alkyl chain having at least 6 and at most 14 carbon atoms. In addition, the second linker has a carboxyl group or an amino group at one end thereof and a thiol group at the other end. When a compound having a molecular weight of 500 or less is used as an electron carrier, the second linker may be an alkyl chain having less than 15 carbon atoms. For example, the length of the alkyl chain of the second linker may be appropriately determined according to the length of the alkyl chain of the first linker and the size (molecular weight) of the electron carrier that is bonded to the first linker.

Next, in the second immobilization step (S2), the redox enzyme is immobilized on the electrode via the second linker. More specifically, as illustrated in (b) in FIG. 5, the second linker immobilized on the electrode and the redox enzyme are bonded to each other. At this time, for example, the amino group of the redox enzyme and the carboxyl group of the second linker are bonded by an amine coupling reaction. Accordingly, the redox enzyme is immobilized on the electrode via the second linker.

### [4. Target molecule redox method]

Next, a target molecule redox method will be described with reference to FIG. 2 and FIG. 6. FIG. 6 is a diagram for describing the target molecule redox method.

The target molecule redox method oxidizes or reduces a target molecule contained in sample 9 by using three-electrode voltage applier 10 including an immobilized enzyme electrode as cathode electrode 1, an anode electrode as counter electrode 3, and reference electrode 2. The surface area of the anode electrode is, for example, sufficiently larger than that of cathode electrode 1.

Sample 9 is an aqueous solution containing a target molecule. The voltage applied to sample 9 may be controlled such that the potential of cathode electrode 1 with respect to reference electrode 2 is the oxidation potential of the electron carrier (electron mediator).

### [Working Examples]

Hereinafter, the target molecule redox method using the immobilized enzyme electrode of the present disclosure will be specifically described with reference to Working Examples, but the present disclosure is not limited only to the following Working Examples in any way.

In Working Example 1 and Comparative Examples 1 to 3, the target molecule reduction rates (for example, residual rate and degradation rate) when an electrode on which nothing was immobilized was used, and when an immobilized enzyme electrode in which at least one of an electron carrier and an redox enzyme was immobilized on the electrode was used were verified.

In Working Example 1 and Comparative Examples 4 and 5, the target molecule reduction rates when the numbers of carbon atoms of the first linker and the second linker are within the respective preferable ranges, and when the number of carbon atoms of the first linker or the second linker is outside the preferable range were verified.

### [Working Example 1]

### (Preparation of sample containing target molecule)

β-lactoglobulin was used as the target molecule. A sample containing the target molecule (hereinafter referred to as the sample) was prepared by dissolving β-lactoglobulin in phosphate-buffered saline (PBS) at pH 7.4 to 3.3 mg/mL.

### (Reduction of target molecule using immobilized enzyme electrode)

A predetermined voltage was applied to the sample at a low temperature for 8 hours using a three-electrode voltage application cell (for example, voltage applier 10 in FIG. 1) and a potentiostat (for example, power supply 20 in FIG. 1). An immobilized enzyme electrode was used as cathode electrode 1 of the three electrodes, an Ag/AgCl electrode was used as reference electrode 2 thereof, and a platinum (Pt) electrode was used as counter electrode 3 thereof. The predetermined voltage applied to the sample containing the target molecule was controlled by a potentiostat such that the potential of cathode electrode 1 with respect to reference electrode 2 was the reduction potential of the electron mediator. In the immobilized enzyme electrode, an alkyl chain having 4 carbon atoms was used as the first linker to immobilize an electron carrier (for example, methylviologen) on the electrode, and an alkyl chain having 10 carbon atoms was used as the second linker to immobilize a redox enzyme (for example, a complex of a thioredoxin reductase and thioredoxin) on the electrode.

### (Confirmation of reduction state of target molecule)

In order to confirm that the target molecule β-lactoglobulin had been reduced, the sample after voltage application was reacted with a digestive enzyme at 37°C for 1 hour, and then subjected to SDS-PAGE. Next, the gel after electrophoresis was stained, and the intensity of the allergenic protein band was quantified by image analysis. As a control sample, β-lactoglobulin before voltage application was also subjected to SDS-PAGE, and the resulting value was taken as a target molecule residual rate of 100%, and the target molecule residual rate of Working Example 1 was calculated by proportional calculation. Electrophoretic patterns are illustrated in FIG. 7. FIG. 7 is a diagram illustrating electrophoretic patterns after SDS-PAGE of Working Example 1, Comparative Example 1, Comparative Example 2, and Comparative Example 3. (a) in FIG. 7 is an electrophoretic pattern of the control sample. (f) in FIG. 7 is an electrophoretic pattern of a molecular weight marker. (e) in FIG. 7 is an electrophoretic pattern of Working Example 1, and a target molecule band was observed at the position surrounded by the dashed line. In Working Example 1, it was confirmed that the residual rate was 70%.

The calculation results of the degradation rates are illustrated in FIG. 8. FIG. 8 is a graph illustrating target molecule degradation rates of Working Example 1, Comparative Example 1, Comparative Example 2, and Comparative Example 3. In Working Example 1, it was confirmed that the target molecule degradation rate was 30%.

### [Comparative Example 1]

The procedure was the same as in Working Example 1, except that a gold electrode (gold electrode in which nothing was immobilized on the electrode) was used as cathode electrode 1. An electrophoretic pattern is illustrated in FIG. 7. (b) in FIG. 7 is an electrophoretic pattern of Comparative Example 1, and a target molecule band was observed in the region surrounded by the dashed line. In Comparative Example 1, it was confirmed that the residual rate was 99%.

In addition, the calculation result of the degradation rate is illustrated in FIG. 8. In Comparative Example 1, it was confirmed that the target molecule degradation rate was 1%.

### [Comparative Example 2]

The procedure was the same as in Working Example 1, except that a gold electrode in which only the electron carrier was immobilized on the electrode was used as cathode electrode 1. An electrophoretic pattern is illustrated in FIG. 7. (c) in FIG. 7 is an electrophoretic pattern of Comparative Example 2, and a target molecule band was observed in the region surrounded by the dashed line. In Comparative Example 2, it was confirmed that the residual rate was 99%.

In addition, the calculation result of the degradation rate is illustrated in FIG. 8. In Comparative Example 2, it was confirmed that the target molecule degradation rate was 1%.

### [Comparative Example 3]

The procedure was the same as in Working Example 1, except that a gold electrode in which only the redox enzyme was immobilized on the electrode was used as cathode electrode 1. An electrophoretic pattern is illustrated in FIG. 7. (d) in FIG. 7 is an electrophoretic pattern of Comparative Example 3, and a target molecule band was observed in the region surrounded by the dashed line. In Comparative Example 3, it was confirmed that the residual rate was 96%.

In addition, the calculation result of the degradation rate is illustrated in FIG. 8. In Comparative Example 3, it was confirmed that the target molecule degradation rate was 4%.

### (Results)

The results of Working Example 1 and Comparative Examples 1 to 3 show that the gold electrode (Comparative Example 1) and the electrode in which only the electron carrier was immobilized on the gold electrode (Comparative Example 2) had a degradation rate of 1%, and that the electrode in which only the redox enzyme was immobilized on the gold electrode (Comparative Example 3) had a degradation rate of 4%. These results are considered to be within the margin of error, and thus it was confirmed that the immobilization of only one of the electron carrier and the redox enzyme on the electrode did not improve the reduction rate of the target molecule. On the other hand, when the immobilized enzyme electrode in which the electron carrier and the redox enzyme were immobilized on the electrode was used (Working Example 1), a significant result of a target molecule degradation rate of 30% was obtained. Therefore, it was confirmed that the target molecule in the sample can be reduced by using the immobilized enzyme electrode according to the present disclosure.

### [Comparative Example 4]

The procedure was the same as in Working Example 1, except that an immobilized enzyme electrode using an alkyl chain having 15 carbon atoms as the second linker was used as cathode electrode 1. As a control sample, β-lactoglobulin before voltage application was also subjected to SDS-PAGE, and the resulting value was taken as a target molecule residual rate of 100%, and the target molecule residual rate of Comparative Example 4 was calculated by proportional calculation, in the same manner as in Working Example 1. Electrophoretic patterns are illustrated in FIG. 9. FIG. 9 is a diagram illustrating electrophoretic patterns after SDS-PAGE of Working Example 1, Comparative Example 4, and Comparative Example 5. (a) in FIG. 9 is an electrophoretic pattern of the control sample. (e) in FIG. 9 is an electrophoretic pattern of a molecular weight marker. (b) in FIG. 9 is an electrophoretic pattern of Comparative Example 4, and a target molecule band was observed at the position surrounded by the dashed line. In Comparative Example 4, it was confirmed that the residual rate was 99%.

The calculation results of the degradation rates are illustrated in FIG. 10. FIG. 10 is a graph illustrating target molecule degradation rates of Working Example 1, Comparative Example 4, and Comparative Example 5. In Comparative Example 4, it was confirmed that the target molecule degradation rate was 1%.

(c) of FIG. 9 is an electrophoretic pattern of Working Example 1, and the residual rate was 72%, and the degradation rate was 28%.

### [Comparative Example 5]

The procedure was the same as in Working Example 1, except that an immobilized enzyme electrode using an alkyl chain having 8 carbon atoms as the second linker was used as cathode electrode 1. An electrophoretic pattern is illustrated in FIG. 9. (d) in FIG. 9 is an electrophoretic pattern of Comparative Example 5, and a target molecule band was observed in the region surrounded by the dashed line. In Comparative Example 5, it was confirmed that the residual rate was 81%.

In addition, the calculation result of the degradation rate is illustrated in FIG. 10. In Comparative Example 5, it was confirmed that the target molecule degradation rate was 19%.

### (Results)

The results of Working Example 1, Comparative Example 4, and Comparative Example 5 demonstrate that when the numbers of carbon atoms of the linkers for immobilizing the electron carrier or the redox enzyme are outside the respective preferable ranges (Comparative Example 4 and Comparative Example 5), this does not improve the target molecule reduction rate. It is considered that because the distance between the electron carrier and the redox enzyme exceeded the electron transferable range, electron transport from the electrode to the redox enzyme was not performed.

On the other hand, it was confirmed that when the numbers of carbon atoms of the linkers for immobilizing the electron carrier and the redox enzyme were within the respective preferable ranges (Working Example 1), the target molecule was efficiently degraded. It is considered that because the distance between the electron carrier and the redox enzyme was within the electron transferable range, electron transport from the electrode to the redox enzyme was performed. For this reason, a significant result of a target molecule degradation rate of 28% was obtained. Therefore, it was confirmed that the target molecule in the sample can be reduced by using the immobilized enzyme electrode according to the present disclosure.

Although the immobilized enzyme electrode, the immobilized enzyme electrode manufacturing method, and the target molecule redox method according to the present disclosure have been described above based on embodiments, the present disclosure is not limited to these embodiments. Various modifications to the foregoing embodiments that can be conceived by those skilled in the art as well as other forms resulting from arbitrary combinations of elements in different embodiments that do not materially depart from the essence of the present disclosure are included within the scope of the present disclosure.

### [Industrial Applicability]

The present disclosure is applicable to an immobilized enzyme electrode that can transport electrons from an electrode to a redox enzyme that is immobilized on the electrode and from the redox enzyme to a target molecule in a sample, and can efficiently oxidize/reduce the target molecule, and to a device that executes a target molecule redox method that uses the immobilized enzyme electrode.

### [Reference Signs List]

- 1: cathode electrode
- 2: reference electrode
- 3: counter electrode
- 4: cell
- 5: lid
- 6a: terminal
- 6b: terminal
- 6c: terminal
- 7a: lead
- 7b: lead
- 7c: lead
- 8: agitating element
- 9: sample
- 10: voltage applier
- 11: glass substrate
- 12: titanium deposition layer
- 13: cathode substrate
- 14: reaction layer
- 20: power supply
- 30: controller
- 40: agitator
- 100: target molecule redox device

## Claims

1. An immobilized enzyme electrode comprising:
an electrode;
a redox enzyme that oxidizes or reduces a target molecule; and
an electron carrier that performs electron transport between the electrode and the redox enzyme, wherein
the electron carrier is immobilized on the electrode via a first linker that is in a form of a chain, and
the redox enzyme is immobilized on the electrode via a second linker that is longer than the first linker, the second linker being in a form of a chain.

2. The immobilized enzyme electrode according to claim 1, wherein
the first linker includes an alkyl chain having at least 2 and at most 5 carbon atoms.

3. The immobilized enzyme electrode according to claim 1 or 2, wherein
the second linker includes an alkyl chain having at least 3 and at most 20 carbon atoms.

4. The immobilized enzyme electrode according to any one of claims 1 to 3, wherein
the electron carrier has a molecular weight of at most 500.

5. An immobilized enzyme electrode manufacturing method for manufacturing the immobilized enzyme electrode according to any one of claims 1 to 4, the immobilized enzyme electrode manufacturing method comprising:
immobilizing the electron carrier on the electrode via the first linker that is in a form of a chain; and
immobilizing the redox enzyme on the electrode via a second linker.

6. A target molecule redox method comprising:
oxidizing or reducing a target molecule using an immobilized enzyme electrode manufactured according to the immobilized enzyme electrode manufacturing method according to claim 5.

7. The target molecule redox method according to claim 6, wherein
the target molecule is a protein having a disulfide bond.

8. A target molecule redox device comprising:
the immobilized enzyme electrode according to any one of claims 1 to 4; and
a power supply that applies voltage to the immobilized enzyme electrode, wherein
the target molecule redox device oxidizes or reduces a target molecule.
